Europäisches Patentamt

European Patent Office

Office européen des brevets

(19)

(11) Veröffentlichungsnummer : **0 027 602**
**B1**

(12) **EUROPÄISCHE PATENTSCHRIFT**

(51) Int. Cl.³ : **A 61 F   5/02**

(45) Veröffentlichungstag der Patentschrift :
**11.04.84**

(21) Anmeldenummer : **80106123.5**

(22) Anmeldetag : **09.10.80**

(54) **Rückenstütz-Bandage.**

(30) Priorität : **19.10.79 DE 2942295**

(43) Veröffentlichungstag der Anmeldung :
**29.04.81 Patentblatt 81/17**

(45) Bekanntmachung des Hinweises auf die Patenterteilung : **11.04.84 Patentblatt 84/15**

(84) Benannte Vertragsstaaten :
**AT BE CH FR GB LI LU NL SE**

(56) Entgegenhaltungen :
**DE-A- 1 566 401**
**DE-A- 2 556 757**
**DE-C-   120 697**
**DE-U- 7 927 415**
**DE-U- 7 929 633**
**FR-A-   806 661**
**FR-A-   874 534**
**FR-A- 1 104 562**
**US-A- 1 327 930**
**US-A- 1 893 960**
**US-A- 2 520 063**
**US-A- 2 541 487**

(73) Patentinhaber : **Annastift e.V. - Forschungslabor-
Helmchenstrasse 1-7**
**D-3000 Hannover 61 (DE)**

(72) Erfinder : **Benning, Herbert**
**Zieglerhof 12**
**D-3000 Hannover 51 (DE)**

(74) Vertreter : **Junius, Walther, Dr.**
**Wolfstrasse 24**
**D-3000 Hannover 81 (DE)**

Rückenstütz-Bandage

Die Erfindung betrifft eine Rückenstützbandage mit einer Pelotte aus einem flachen, federnden Material, welches im entspannten Zustand eine dem Rücken des Trägers zugekehrte Wölbung aufweist.

Derartige Rückenstütz-Bandagen dienen zur Stützung und Korrektur der menschlichen Wirbelsäule. Sie bestehen aus einer fest um den Leib spannbaren elastischen Bandage, die an ihrer Innenseite einen symmetrisch beiderseits neben der Wirbelsäule am Rücken zur Anlage kommenden Stützkörper trägt.

Es sind mehrere Rückenstütz-Bandagen bekannt geworden.

Eine Rückenstütz-Bandage mit einer federnden, plattenförmigen Pelotte ist aus der DE-OS 25 56 757 bekannt geworden. Zur Abstützung dieser Pelotte sind in der Bandage vertikale Stäbe einzuarbeiten, auf die sich am oberen und am unteren Ende die im Längsschnitt gewölbte Pelotte abstützt. Diese Pelotte kann auch in der Mitte einen vertikalen Schlitz aufweisen, um nicht auf die Wirbelsäule zu drücken. Diese Rückenstützbandage hat sich bewährt, bezweckt aber eine Abstützung des ganzen Bereichs der Lendenwirbelsäule und stellt damit unter Umständen eine Überversorgung des Patienten dar, und zwar dann, wenn nur der Übergang zwischen Kreuzbein und Lendenwirbelsäule zu versorgen ist.

Durch die DE-GM 79 27 415 ist eine starre Pelotte von Herzform bekannt geworden, die gewölbt ist, wobei die Wölbung dem Rücken des Trägers abgewendet ist. Solche starren Pelotten bereiten dem Träger beim Tragen Schwierigkeiten und sind wenig wirksam, weil von den sie tragenden Bandagen kein Druck auf den Bereich seitlich des Kreuzbeines gebracht werden kann.

Die Rückenstütz-Bandage nach der GB-PS 909 970 besteht aus drei Längsbändern, an welchen eine Vielzahl von zueinander parallelen Leisten befestigt ist. Diese Leisten kommen beim Anlegen der Bandage auf dem menschlichen Rücken zu beiden Seiten neben der Wirbelsäule zu liegen. Die von oben nach unten durchlaufenden, ungeteilten Leistn sind jedoch nicht befriedigend in der Lage, die Muskeln der einzelnen Wirbelknochen zu bearbeiten und Rückgratbewegungen des Gurtträgers mitzumachen. Auch tragen diese Leisten unter der Bandage erheblich auf, so daß von außen eine derartige angelegte Bandage sogleich unter der Kleidung erkennbar ist.

Der Gurt der DE-PS 578 241 zur Stützung des Kreuz- und Darmbeines sowie der Lendenwirbelsäule ist mit zwei sich kurezenden Bandfedern versehen, welche an ihren Endpunkten einen am Rücken zur Anlage kommenden Stützkörper tragen. Über die gekreuzten Bandfedern sind zwei Gurte geführt, welche um den Leib gespannt werden und die zwei übereinander angeordnete Paare von Stützkörpern symmetrisch beiderseits neben der Wirbelsäule auf den menschlichen Rücken drücken. Diese Rückenstütz-Bandage vermag die menschliche Wirbelsäule in ihrem primär gefährdeten Bereich, nämlich im Bereich der Lendenwirbel und des Kreuzbeines, nicht optimal wirksam zu stützen. Da die vier Stützkörper eine relativ große Auflagefläche am Rücken haben, muß dieser Bandage sehr straff gespannt sein, damit die Stützkörper genügend fest angedrückt werden, um ihre Wirkung zu entfalten. Das Tragen dieser Rückenstützbandage ist wegen ihrer erheblichen Dimensionen von außen sofort erkennbar.

Durch die DE-PS 18 16 588 ist eine weitere Rückenstütz-Bandage bekannt geworden, bei der jeweils zwei auf gleicher Höhe liegende Stützkörper durch ein brückenförmiges Element miteinander verbunden sind und damit eine Einheit bilden, wobei mehrere dieser Einheiten senkrecht übereinander angeordnet sind. Die brückenförmigen Elemente sind an ihren Scheitelpunkten an einer mittleren senkrechten Schiene drehbar angeordnet. Die die Stützkörper, die ihrerseits schon eine erhebliche Dicke aufweisen, verbindende Brücke und deren Befestigung an einer senkrechten Schiene ergeben ein Bauteil von erheblicher Dicke, dessen Tragen unter der Kleidung sofort von außen auffällt und sichtbar ist.

Es ist die Aufgabe der Erfindung, eine nur sehr gering auftragende Rückenstütz-Bandage zu schaffen, die eine hervorragende Stützung und Entlastung der Wirbelsäule bei statisch muskulärer Insuffizienz und Störungen im Ilio-Sakralbereich schafft.

Die Erfindung besteht darin, daß die Pelotte mit Wölbung im horizontalen Querschnitt und so ausgebildet ist, daß sie im gespannten Zustand im Querschnitt in eine Wellenlinie übergeht, die aus drei Wölbungen besteht, einer mittleren Wölbung, die dem Rücken des Trägers abgekehrt ist und zwei seitlich daneben liegenden Wölbungen, die dem Rücken des Trägers zugekehrt sind.

Eine derartige Rückenstütz-Bandage trägt nur äusserst gering auf. Denn es ist ja nur das Material der Bandage und die aus einer federnden Kunststoffplatte oder einem dünnen Metallblech hergestellte Pelotte, die zusammen nur wenige Millimeter auftragen, so daß das Tragen dieser Rückenstützbandage unter der Kleidung nicht oder nur in sehr geringem Umfange erkennbar ist. Dabei wird durch das Zusammenwirken der plattenförmig ausgebildeten, als ein einziges Stück gefertigten Pelotte und den von der Bandage auf die als Hebel wirkenden seitlichen Randbereiche der Pelotte ausgeübten Kräfte eine hervorragend gute Stützwirkung erzielt.

Vorteilhaft ist es, wenn im Randbereich zusätzlich zu den im gespannten Zustand auftretenden drei Wölbungen noch je eine der Körperseite zugekehrte Biegung vorgesehen ist. Diese bildet dann eine Abstützfläche für die Bandage in einer Weise, daß sich der Rand der Pelotte nicht durch die Bandage abzeichnet.

Zweckmäßig ist es, wenn der vertikale Querschnitt der als Pelotte dienenden Platte im mittleren Bereich die anatomische Außenform der Kreuzbeingegend aufweist.

Besonders vorteilhaft ist es, wenn die als Pelotte dienende Platte mindestens zwei quer verlaufende Schlitze aufweist, weil dann der Übergang des Plattenmateriales in die drei Wölbungen bei Spannung dieses Materiales besonders leicht vor sich geht. Zur Erzielung der gewünschten Abstützung der beiden äußeren Wölbungen der Pelotte auf Flächen neben der Wirbelsäule ist es zweckmäßig, wenn die Schlitze zwei Verengungen im Bereich der beiden äußeren Wölbungen aufweisen. Diese Verengungen der Schlitze führen auch zu einer besonders gut ausgebildeten, für die Abstützung notwendigen Form der Pelotte.

Zweckmäßig ist es, wenn die Pelottenplatte in einem schweißaufsaugenden Bezug untergebracht ist. In einfacher Weise läßt sich die Pelotte oder ihr Bezug mit einem Klettenverschluß in der Bandage befestigen.

Das Wesen der Erfindung ist nachstehend anhand eines in der Zeichnung schematisch dargestellten Ausführungsbeispieles näher erläutert. Es zeigen :

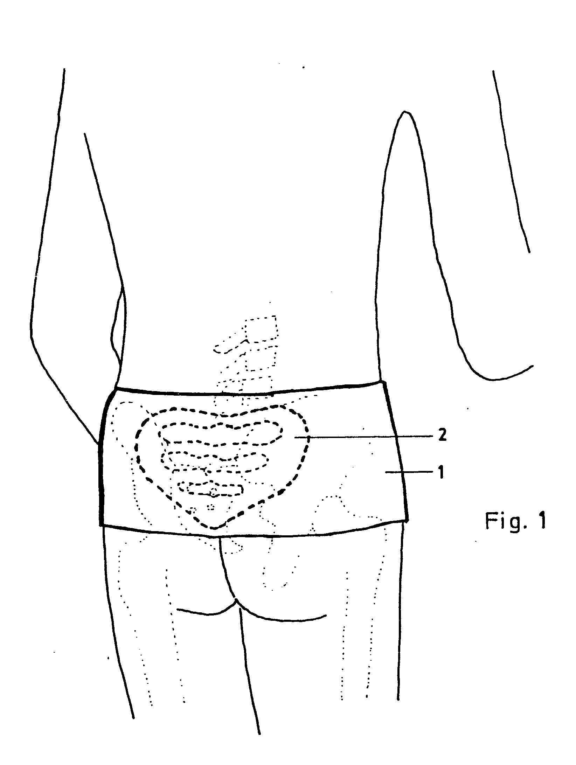

Figur 1 eine Ansicht der Rückenstütz-Bandage, am Menschen angelegt,

Figur 2 eine Ansicht der Pelotte,

Figur 3 eine Ansicht der Rückenstütz-Bandage mit Pelotte von schräg oben,

Figur 4 einen Querschnitt durch die Pelotte im ungespannten Zustand,

Figur 5 einen Querschnitt durch die Pelotte im gespannten Zustand,

Figur 6 einen Querschnitt durch die Rückenstütz-Bandage während des Anlegens vor dem Spannen der Pelotte,

Figur 7 einen Querschnitt durch die an den menschlichen Körper angelegte Rückenstütz-Bandage mit gespannter Pelotte.

Die Rückenstütz-Bandage besteht aus einer um den Körper zu legenden Bandage und der aus einem flachen, federnden Material hergestellten Pelotte 2, die in einem Bezug untergebracht ist, der mit einem Klettenverschluß in der Bandage befestigt ist. Die Pelotte 2 ist zusätzlich noch mit einem Polster versehen.

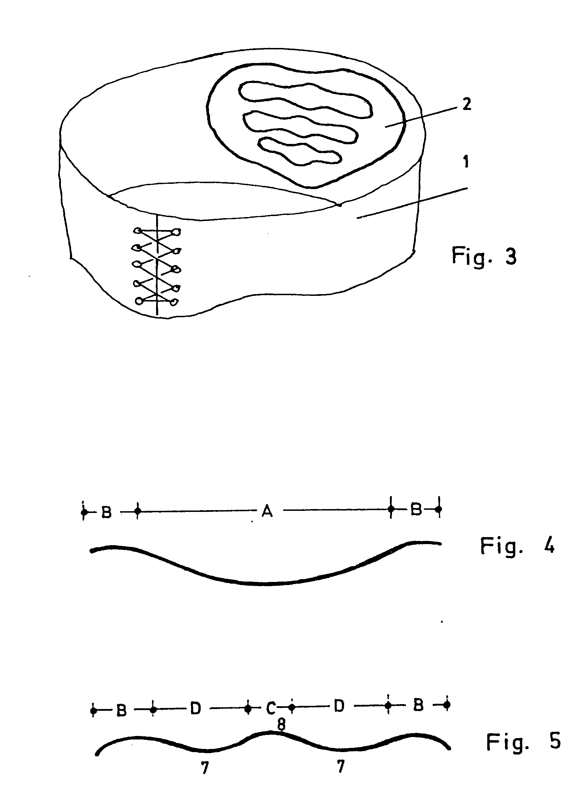

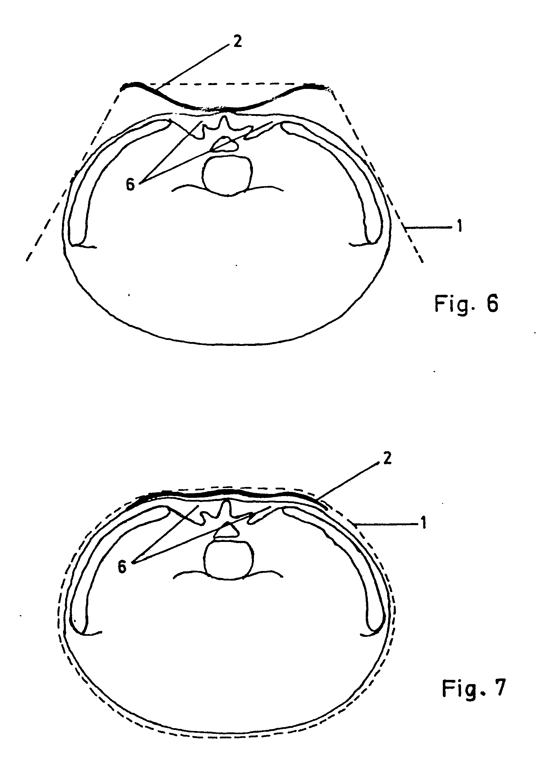

Wesentliches Teil dieser Rückenstütz-Bandage ist die Pelotte 2, die aus einem Stück federnden Kunststoffes oder aus einem Stück federnden, plattenförmigen Bleches hergestellt ist. Die Pelotte 2 weist im ungespannten Zustand eine Wölbung auf, die dem Rücken des Trägers zugekehrt und damit der Rückenseite (Innenseite) der Bandage abgekehrt ist. Diese Wölbung ist in Fig. 4 in dem Bereich A vorhanden. Die Ränder sind in den Randbereichen B zusätzlich in gleicher Richtung gebogen, um eine breite, flächenhafte Abstützung für die Bandage 1 zu bieten. Wird nun der Gurt am menschlichen Körper gespannt, so wird die Wölbung der Pelotte unter dieser Spannung deformiert. Dabei geht diese Wölbung im gespannten Zustand im Querschnitt in eine Wellenlinie über, die aus drei Wölbungen besteht : Einer mittleren Wölbung im Bereich C, die dem Rücken des Trägers abgekehrt ist und zwei seitlich daneben liegenden Wölbungen im Bereich D, die dem Rücken des Trägers zugekehrt sind. Diese drei Wölbungen befinden sich innerhalb der Randbereiche B, die der Körperseite der Bandage zugekehrt gebogen sind. Durch diese gut aus Fig. 5 und 7 erkennbare Biegung der gewölbten Pelotte ist, wie man besonders gut aus Fig. 7 ersehen kann, der Pelotte eine Form gegeben, bei der die Wölbungen im Bereich D sich auf der Muskulatur neben der Wirbelsäule 6 abstützen. Diese Wölbungen 7 kommen somit beiderseits neben der Wirbelsäule am Rücken des Trägers zur Anlage, während die Wölbung 8 in der Mitte zwischen diesen beiden Wölbungen nach außen, also vom Rücken des Trägers weg gedrückt ist, so daß auf den Dornfortsätzen der Wirbelsäule selbst keine Abstützung der Pelotte erfolgt.

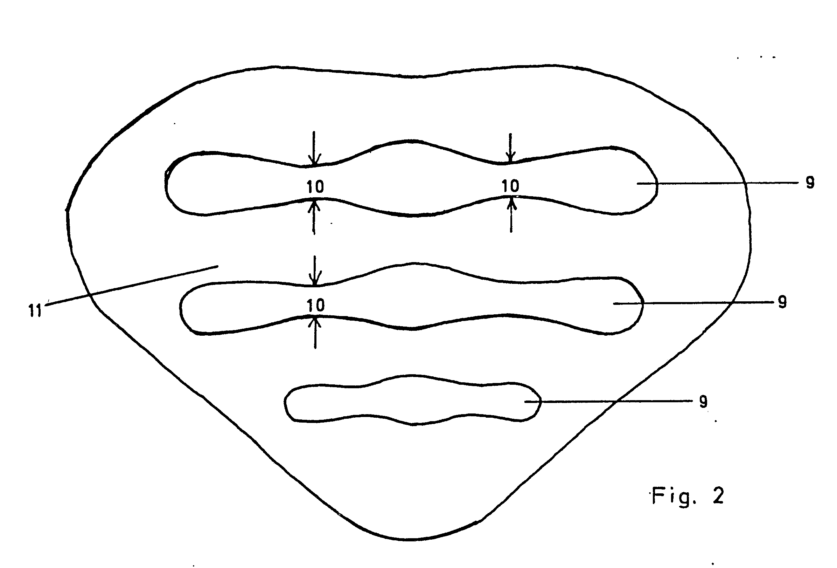

Wie aus Fig. 2 ersichtlich ist, weist die Pelotte im wesentlichen an ihrem Umfang eine Herform auf. Diese Form gewährleistet eine gute Abstützung und ein ungestörtes Tragen der Rückenstütz-Bandage. In Fig. 2 sind mehrere Schlitze 9 ersichtlich, die sich in der Pelotte befinden. Diese Schlitze verlaufen quer zur Richtung der Wirbelsäule. Die Schlitze weisen dort, wo sich die seitlichen Wölbungen 7 ausbilden, Verengungen 10 auf, die für die dazwischenliegenden Bereiche 11 breite Flächen erbringen, die sich dann wirkungsvoll auf die A Muskelbereiche neben der Wirbelsäule stützen und dadurch die Stützwirkung dieser Rückenstütz-Bandage bewirken.

## Ansprüche

1. Rückenstütz-Bandage mit einer Pelotte aus einem flachen, federnden Material, welches im entspannten Zustand eine dem Rücken des Trägers zugekehrte Wölbung aufweist, dadurch gekennzeichnet, daß die Pelotte (2) mit Wölbung im horizontalen Querschnitt und so ausgebildet ist, daß sie im gespannten Zustand im Querschnitt in eine Wellenlinie übergeht, die aus drei Wölbungen (7, 8, 7) besteht, einer mittleren Wölbung (8), die dem Rücken des Trägers abgekehrt ist und zwei seitlich daneben liegenden Wölbungen (7), die dem Rücken des Trägers zugekehrt sind.

2. Rückenstütz-Bandage nach Anspruch 1, dadurch gekennzeichnet, daß die Pelotte (2) zumindest zwei horizontal verlaufende Schlitze (9) aufweist.

3. Rückenstütz-Bandage nach Anspruch 2, dadurch gekennzeichnet, daß die Schlitze (9) zwei Verengungen (10) im Bereich der beiden äußeren Wölbungen (7) aufweisen.

4. Rückenstütz-Bandage nach Anspruch 1, dadurch gekennzeichnet, daß im Randbereich der Pelotte (2) je eine der Körperseite zugekehrte Biegung vorgesehen ist.

5. Rückenstütz-Bandage nach Anspruch 4, dadurch gekennzeichnet, daß der vertikale Querschnitt der Pelotte (2) im mittleren Bereich die anatomische Außenform der Kreuzbeingegend aufweist.

6. Rückenstütz-Bandage nach Anspruch 1, dadurch gekennzeichnet, daß der Außenrand der Pelotte (2) Herzform aufweist.

7. Rückenstütz-Bandage nach Anspruch 1, dadurch gekennzeichnet, daß die Pelotte (2) aus federndem Kunststoff oder federndem Blech hergestellt ist.

8. Rückenstütz-Bandage nach Anspruch 1, dadurch gekennzeichnet, daß die Pelotte (2) in einem schweißaufsaugenden Bezug untergebracht ist.

9. Rückenstütz-Bandage nach Anspruch 1, dadurch gekennzeichnet, daß die Pelotte (2) mit einem Polster versehen ist.

10. Rückenstütz-Bandage nach Anspruch 1, dadurch gekennzeichnet, daß die Pelotte (2) oder ihr Bezug mit einem Klettenverschluß in der Bandage befestigt ist.

## Claims

1. A dorsal-supporting bandage having a pressure pad of a flat, springy material which in the relaxed state has a curvature facing towards the back of the wearer, characterised in that the pressure pad (2) has a curvature in horizontal cross-section and is so constructed that in the stressed state, it changes into a wavy line in cross-section, which wavy line consists of three curves (7, 8, 7), a central curve (8), which faces away from the back of the wearer, and two curves (7) close thereto on either side which face towards the back of the wearer.

2. A dorsal-supporting bandage according to Claim 1, characterised in that the pressure pad (2) has at least two horizontally-extending slots (9).

3. A dorsal-supporting bandage according to Claim 2, characterised in that the slots (9) have two constrictions (10) in the region of the two outer curves (7).

4. A dorsal-supporting bandage according to Claim 1, characterised in that in the edge region of the pressure pad (2), a respective bend facing towards the side of the body is provided.

5. A dorsal-supporting bandage according to Claim 4, characterised in that the vertical cross-section of the pressure pad (2) in the central region exhibits the anatomical outer shape of the sacral region.

6. A dorsal-supporting bandage according to Claim 1, characterised in that the outer edge of the pressure pad (2) is heart-shaped.

7. A dorsal-supporting bandage according to Claim 1, characterised in that the pressure pad (2) is produced from an elastic synthetic resin or a springy metal sheet.

8. A dorsal-supporting bandage according to Claim 1, characterised in that the pressure pad (2) is accommodated in a sweat-absorbing cover.

9. A dorsal-supporting bandage according to Claim 1, characterised in that the pressure pad (2) is provided with a cushion.

10. A dorsal-supporting bandage according to Claim 1, characterised in that the pressure pad (2), or its cover, is fixed to the bandage by means of a burr-type fastening.

## Revendications

1. Bandage de support dorsal comprenant une pelote faite d'une matière élastique plate qui, à l'état détendu, présente une cambrure dirigée vers le dos du porteur, caractérisé en ce que la pelote (2) est munie d'une cambrure dans la section horizontale et est configurée de manière qu'à l'état tendu, elle prenne en section la forme d'une ligne ondulée qui est composée de trois cambrures (7, 8, 7), une cambrure centrale (8), dirigée dans le sens qui s'éloigne du dos du porteur et deux cambrures (7) latéralement adjacentes à la première et qui sont dirigées vers le dos du porteur.

2. Bandage de support dorsal selon la revendication 1, caractérisé en ce que la pelote (2) présente au moins deux fentes (9) s'étendant horizontalement.

3. Bandage de support dorsal selon la revendication 2, caractérisé en ce que les fentes (2) présentent deux rétrécissements (10) dans la région des deux cambrures extérieures (7).

4. Bandage de support dorsal selon la revendication 1, caractérisé en ce qu'il est prévu dans chacune des régions latérales de la pelote (2), un cintrage dirigé vers le côté du corps.

5. Bandage de support dorsal selon la revendication 4, caractérisé en ce que la section verticale de la pelote (2) présente dans la région centrale la forme anatomique de la région du sacrum.

6. Bandage de support dorsal selon la revendication 1, caractérisé en ce que le bord extérieur de la pelote (2) présente la forme d'un cœur.

7. Bandage de support dorsal selon la revendication 1, caractérisé en ce que la pelote (2) est faite d'une matière plastique élastique ou d'une tôle élastique.

8. Bandage de support dorsal selon la revendication 1, caractérisé en ce que la pelote (2) est contenue dans un habillage capable d'absorber la transpiration.

9. Bandage de support dorsal selon la revendication 1, caractérisé en ce que la pelote (2) est munie d'un rembourrage.

10. Bandage de support dorsal selon la revendication 1, caractérisé en ce que la pelote (2) ou son habillage est fixée dans le bandage au moyen d'une fixation à peluche et crochets.

Fig. 1

0 027 602

Fig. 2

Fig. 3

Fig. 4

Fig. 5

Fig. 6

Fig. 7